# EUROPEAN PATENT APPLICATION

(11) **EP 1 867 334 A1**
(43) Date of publication of application: **19.12.2007**
(21) Application number: 06713975.8
(22) Date of filing: 17.02.2006
(51) Int. Cl.: A61K 31/573, A61K 9/14, A61K 47/34, C07J 5/00

(54) **METHOD OF RELIEVING OR AVOIDING SIDE EFFECT OF STEROID COMPOUND**

(30) Priority: 18.02.2005 JP 2005041690
(71) Applicant: SANTEN PHARMACEUTICAL CO., LTD., Higashiyodogawa-ku, Osaka-shi, Osaka 533-8651 (JP)
(72) Inventor: YAMADA, Kazuhito, SANTEN PHARMACEUTICAL CO., LTD., Osaka-shi, Osaka 5338651 (JP); OOHASHI, Tokie, Santen Pharmaceutical Co., Ltd., Ikoma-shi, Nara 6300101 (JP); SAKAI, Hiroyuki, Santen Pharmaceutical Co., Ltd., Ikoma-shi, Nara 6300101 (JP); MATSUNO, Kiyoshi, Santen Pharmaceutical Co., Ltd., Ikoma-shi, Nara 6300101 (JP); KIMURA, Akio, SANTEN PHARMACEUTICAL CO., LTD., Osaka-shi, Osaka 5338651 (JP)
(74) Representative: Müller-Boré & Partner Patentanwälte
(86) International application number: PCT/JP2006/302834
(87) International publication number: WO 2006/088134

(57) **Abstract**

The invention provides a method of relieving or avoiding a steroid-induced increase in intraocular pressure caused by the administration of a steroid. The steroid-induced increase in intraocular pressure can be relieved or avoided by incorporating a steroid in fine particles.

## Description

### Technical Field

The present invention relates to a method of relieving or avoiding a steroid-induced increase in intraocular pressure by incorporating a steroid in fine particles and a composition therefor.

### Background Art

Steroids are widely used as therapeutic agents for various inflammatory diseases. Examples of steroids to be used in an ophthalmic field include betamethasone, dexamethasone, triamcinolone, fluorometholone, fluocinolone acetonide and the like. Such a steroid is a very useful agent. However, it is known that a steroid has a side effect of a steroid-induced increase in intraocular pressure. The side effect may be manifested by an increase in intraocular pressure when, for example, a steroid is continuously administered to a patient or it is administered to a steroid-sensitive patient. When it is serious, irreversible impairment of visual function is known to be caused.
Therefore, in the case of administering a steroid, an ophthalmologist has to keep the side effect in mind. However, because there are many unclear points on the mechanism of onset or pathological conditions of the side effect, it is considered to be very difficult to predict the onset of the side effect. At present, when the steroid-induced increase in intraocular pressure is caused, generally an ophthalmologist first discontinues the administration of the steroid. Therefore, there are problems that due to the side effect, the useful drug efficacy of a steroid cannot be utilized, and a sufficient treatment cannot be provided.

On the other hand, as means for suppressing a steroid-induced increase in intraocular pressure, use of a drug such as an intraocular pressure lowering agent (Exp. Eye Res. , 54, 211-218, 1992, JP-A-2004-256524) or a surgical technique such as trabeculectomy is known. However, a technique for suppressing a steroid-induced increase in intraocular pressure without using such means has not been known.

Thus, it is an interesting subject to relieve or avoid a side effect of a steroid-induced increase in intraocular pressure upon administration of a steroid without using a drug such as an intraocular pressure lowering agent. Further, it is an important subject to more effectively utilize the excellent effect of a steroid.

### Disclosure of the Invention

### Problems to be Solved

That is, an object of the invention is to provide a method capable of relieving or avoiding a side effect of a steroid-induced increase in intraocular pressure upon administration of a steroid to sufficiently utilize the excellent effect of the steroid without using a drug such as an intraocular pressure lowering agent.

### Means of Solving Problems

The present inventors made intensive studies and as a result, they found a method capable of relieving or avoiding a steroid-induced increase in intraocular pressure without using a drug such as an intraocular pressure lowering agent by incorporating a steroid in fine particles even if an ophthalmic composition containing the steroid was used.

It has been said that sub-Tenon's administration, subconjunctival administration or intravitreal administration is preferred for applying a steroid to diseases of the back of the eye. However, in any of these administration methods, a steroid is injected into ocular tissues and allowed to stay in the tissues for a long time, therefore, an increase in intraocular pressure is a more serious problem. In the invention, it was found that the increase in intraocular pressure can be relieved or avoided by using an ophthalmic composition in which a steroid is incorporated in fine particles particularly in the case where such a steroid is administered to the sub-Tenon. According to the invention, a steroid-induced increase in intraocular pressure can be relieved or avoided, therefore, the excellent drug efficacy of a steroid can be sufficiently utilized without discontinuing the administration of steroid.

That is, the invention relates to
(1) a method of relieving or avoiding a steroid-induced increase in intraocular pressure by incorporating a steroid in an ophthalmic composition in fine particles;
(2) the method according to the above (1), wherein the site of administration is the sub-Tenon;
(3) the method according to the above (2), wherein the dosage form of the ophthalmic composition is an injection;
(4) the method according to the above (3), wherein an average particle diameter of the fine particles is 50 nm to 150 µm;
(5) the method according to the above (4), wherein the fine particles are made of a biodegradable or biosoluble polymer;
(6) the method according to the above (3), wherein the biodegradable or biosoluble polymer is polylactic acid or poly(lactic acid-glycolic acid);
(7) the method according to the above (1), wherein the steroid is betamethasone, dexamethasone, triamcinolone, prednisolone, fluorometholone, hydrocortisone or fluocinolone acetonide; and
(8) an ophthalmic composition comprising a steroid, wherein a steroid-induced increase in intraocular pressure is relieved or avoided by incorporating the steroid in fine particles.

The invention also relates to a method of relieving or avoiding a steroid-induced increase in intraocular pressure comprising administering an effective amount of an ophthalmic composition containing fine particles incorporating a steroid to a patient.

In the invention, as a material for forming the fine particles, a biodegradable or biosoluble polymer is preferred. Specific examples thereof include biodegradable polymers such as polylactic acid, poly(lactic acid-glycolic acid), polylactic acid-polyethyleneglycol block copolymers, polylactic acid-polyethyleneglycol-polylactic acid block copolymers, poly(lactic acid-glycolic acid)-polyethyleneglycol block copolymers, poly(lactic acid-glycolic acid)-polyethyleneglycol-poly(lactic acid-glycolic acid) block copolymers, lactic acid-caprolactone copolymers, polyanhydrides, polyorthoesters, poly-epsilon-caprolactone, polyacrylcyanoacrylates, polyhydroxyalkanoates, polyphosphoesters, and poly-α-hydroxyacids; natural polymers such as gelatin, dextran, albumin and chitosan; and synthetic polymers such as methacrylic acid copolymers and poly-N-alkylacrylamide.

The molecular weight of these polymeric substances is not particularly limited and can be appropriately selected depending on the kind of the steroid to be incorporated in the fine particles, the effective therapeutic concentration of the steroid, the release period of the steroid or the like.

The particle diameter of the fine particles according to the invention is preferably 50 nm to 150 µm. It is difficult to produce fine particles having a particle diameter of 50 nm or less, and fine particles having a particle diameter of 150 µm or more are too large and are not preferred to be used in the form of an injection. A more preferred particle diameter is 200 nm to 80 µm.

Examples of the fine particles with a micrometer order in which a steroid is incorporated include microspheres, and examples of the fine particles with a nanometer order include nanospheres.

The ophthalmic composition of the invention is preferably used for treatment or prevention of diseases of a retina, a choroid and an optic nerve. Specific examples of the disease include inflammation due to various causes, viral or bacterial infections, diseases due to angiogenesis of a retina-choroid, diseases due to ischemia of a retina and optic nerve disorders due to glaucoma. More specific examples of the disease include uveitis, cytomegalovirus retinitis, macular edema, age-related macular degeneration, neovascular maculopathy, diabetic retinopathy, idiopathic macular pucker, proliferative vitreoretinopathy, retinal detachment, retinitis pigmentosa, central retinal vein occlusion, central retinal artery occlusion, branch retinal vein occlusion, branch retinal artery occlusion and the like.

In the invention, the kind of the steroid is not particularly limited, however, examples thereof include betamethasone, dexamethasone, prednisolone, methylprednisolone, fluorometholone, triamcinolone, hydrocortisone, beclomethasone, fluocinolone acetonide, progesterone and the like, and a more preferred steroid is betamethasone, dexamethasone or fluocinolone acetonide. A salt of the steroid of the invention is not particularly limited as long as it is a pharmaceutically acceptable salt, and examples thereof include sodium salts, potassium salts and the like. Further, an ester of the steroid of the invention is also not particularly limited as long as it is a pharmaceutically acceptable ester, and examples thereof include acetate esters, phosphate esters, (metasulfo) benzoate esters, maleate esters, formate esters, valerate esters, propionate esters and the like.

A preferred form of the fine particles incorporating a steroid is a matrix-type in which a drug (a steroid) is dispersed uniformly in the fine particles or a capsule-type in which a drug as a core is encapsulated in the fine particles.

An amount of the drug to be incorporated in the fine particles can be appropriately increased or decreased depending on the kind of the drug, the effective therapeutic concentration of the drug, the release period of the drug, symptoms of diseases or the like. The content of the drug is 0.01 to 95% by weight, preferably 0.1 to 20% by weight of the fine particles.

The fine particles according to the invention can be produced by a grinding method using a mill, a phase separation method (a coacervation method), a spray drying method, a supercritical fluid method, an interfacial deposition method or an interfacial reaction method, which is known, however, the method is not limited to them. More specific examples of the method include a solvent evaporation method which is an interfacial deposition method (J. Control. Release, 2, 343-352, (1985)), an interfacial polymerization method which is an interfacial reaction method (Int. J. Pharm., 28, 125-132 (1986)), a self-emulsification solvent diffusion method (J. Control. Release,25, 89-98 (1993)) and the like. An appropriate production method can be arbitrarily selected among these production methods considering the particle diameter of the fine particles, the kind, properties or content of the drug to be incorporated or the like.

As a specific production example of the fine particles, a production example of drug-containing fine particles will be shown in Examples described below in which betamethasone is used as the steroid and polylactic acid is used as the material of the fine particles.

Examples of the administration method of the ophthalmic composition of the invention include sub-Tenon's administration, subconjunctival administration, intravitreal administration and the like, and sub-Tenon's administration is particularly preferred. The sub-Tenon's administration can be carried out by employing a conventional sub-Tenon's injection.

The dosage form of the ophthalmic composition of the invention is preferably an injection. The injection can be prepared by employing widely used formulation techniques of injections. For example, a preparation can be prepared by adding a commonly used additive such as an osmotic pressure adjusting agent such as sodium chloride, a buffer such as sodium phosphate, a surfactant such as polysorbate 80 or a viscous agent such as methyl cellulose and the fine particles to distilled water for injection. When a high pressure syringe with no needle is used, the fine particles can be administered, as they are, without formulating them into an injection.

The dose of the steroid varies depending on the kind of the steroid. However, it is generally about 1 µg to 100 mg at one time (administration frequency may be once to several times per day to once per several months), and can be increased or decreased according to the patient's age, symptoms or the like.

### Advantage of the Invention

As will be described in detail in the section of Examples below, in a pharmacological test, when a suspension of betamethasone was administered to the sub-Tenon, a steroid-induced increase in intraocular pressure was observed. However, when a suspension of betamethasone-loaded fine particles was administered to the sub-Tenon, a steroid-induced increase in intraocular pressure was not observed. That is, the invention provides a method of relieving or avoiding a steroid-induced increase in intraocular pressure by incorporating a steroid in fine particles, and a composition therefor.

### Best Mode for Carrying Out the Invention

Hereinafter, a production example of fine particles, a pharmacological test, and a preparation example will be described, however, these examples are described for the purpose of understanding the invention better and are not meant to limit the scope of the invention.

### 1. Production of Steroid-loaded Fine Particles Production Example 1

Betamethasone (0.05 g) and polylactic acid (0.25 g) having a weight average molecular weight of about 20,000 (degree of dispersion: about 2.0) were dissolved in dichloromethane (0.5 ml) and benzyl alcohol (3.0 ml), and the obtained solution was used as a drug/polymer solution. A 0.2% (w/v) aqueous polyvinyl alcohol solution (400 ml) was homogenized with a homogenizer (10,000 rpm), and the drug/polymer solution was added dropwise to the homogenized solution. The mixture was homogenized for 10 minutes after completion of dropwise addition thereby preparing an O/W emulsion. The O/W emulsion was stirred (200 rpm) for 3 hours with a stirrer. After completion of stirring, the obtained suspension was centrifuged, and the resulting supernatant was removed. In order to wash the precipitate, ultrapure water (30 ml) was added to disperse the precipitate, and the resulting dispersion was centrifuged again, and the resulting supernatant was removed. This procedure was carried out one more time. The washed precipitate was sieved thereby obtaining particles. The obtained particles were lyophilized, whereby betamethasone-loaded microspheres having a particle diameter of 2 µm to 70 µm and a betamethasone content of 11.6% were obtained.

### 2. Pharmacological Test

In order to examine an effect of incorporation of a steroid in fine particles on avoiding a steroid-induced increase in intraocular pressure, the composition of the invention was administered to the sub-Tenon of a cat (strain: Eur., sex: male), and a test for an effect on avoiding a steroid-induced increase in intraocular pressure was carried out.

### (Preparation of Test Compound-Containing Liquid)

The betamethasone-loaded microspheres obtained in Production Example 1 were suspended in a solvent (an aqueous solution containing 5% (w/v) mannitol, 0.1% (w/v) polysorbate 80 and 0.5% (w/v) sodium carboxymethyl cellulose), whereby suspensions containing 8.6% (w/v) and 25.8% (w/v) betamethasone-loaded microspheres (hereinafter referred to as BMMS suspension 1 and BMMS suspension 2, respectively) were prepared, respectively.

### (Preparation of Comparative Control Liquid)

As controls, a suspension of betamethasone (hereinafter referred to as BM suspension) and a suspension of betamethasone-unloaded microspheres (hereinafter referred to as PLA-MS suspension) were prepared. As for the BM suspension, a 6.0% (w/v) BM suspension was prepared by suspending betamethasone in a solvent (an aqueous solution containing 5% (w/v) mannitol, 0.1% (w/v) polysorbate 80 and 0.5% (w/v) sodium carboxymethyl cellulose). As for the PLA-MS suspension, a 25.8% (w/v) PLA-MS suspension was prepared by suspending betamethasone-unloaded microspheres obtained in the same manner as in Production Example 1 except that betamethasone was not used in a solvent (an aqueous solution containing 5% (w/v) mannitol, 0.1% (w/v) polysorbate 80 and 0.5% (w/v) sodium carboxymethyl cellulose).

### (Administration Method and Measurement Method)

According to the following method, the intraocular pressure was measured in animal groups administered with BMMS suspension 1 and BMMS suspension 2, respectively (BMMS administration group 1 and BMMS administration group 2), an animal group administered with BM suspension (BM administration group) and an animal group administered with PLA-MS suspension (PLA-MS administration group).
1) Cats (strain: Eur., sex: male) were given systemic anesthesia, and a solution of Benoxil (0.1% (w/v)) was instilled into both eyes thereby anesthetizing the ocular surface.
2) The bulbar conjunctiva was incised to expose the Tenon's capsule, and BMMS suspension 1 was administered to the sub-Tenon in an amount of 100 µl per eye using a 24G sub-Tenon' s anesthesia needle. Further, BMMS suspension 2, BM suspension and PLA-MS suspension were also administered in an amount of 100 µl per eye, respectively.

The intraocular pressure was measured with an applanation tonometer over 4 weeks, and a difference with the intraocular pressure just before the administration was calculated, and then, comparison among BMMS administration group 1, BMMS administration group 2, BM administration group and PLA-MS administration group was carried out.

### (Results and Discussion)

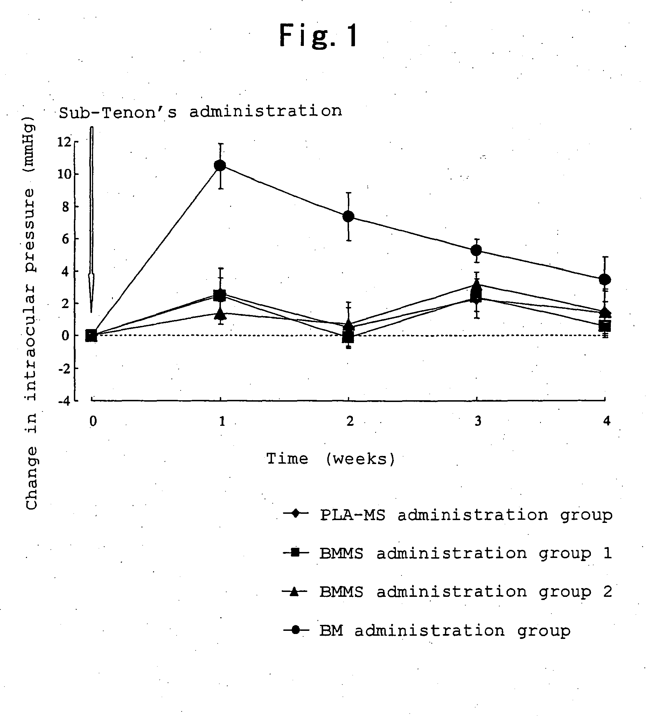

The results of the test for an effect on avoiding an increase in intraocular pressure using cats are shown in Fig. 1. The value of the change in intraocular pressure in the graph represents a value (average) changed from the initial intraocular pressure. Incidentally, as for the number of cases, BM administration group includes 8 eyes, and BMMS administration group 1, BMMS administration group 2 and PLA-MS administration group include 10 eyes, respectively. As is apparent from Fig. 1, in sub-Tenon's administration of betamethasone to cats, by incorporating betamethasone in microspheres, a steroid-induced increase in intraocular pressure can be relieved or avoided.

### 3. Preparation Example

| | | |
|---|---|---|
| BMMS Suspension 1 (in 100 ml) | | |
| | Betamethasone-loaded microspheres | 8.6 g |
| | Mannitol | 5 g |
| | Polysorbate 80 | 0.1 g |
| | Sodium carboxymethyl cellulose | 0.5 g |
| | Sterile purified water | q.s. |
| BMMS Suspension 2 (in 100 ml) | | |
| | Betamethasone-loaded microspheres | 25.8 g |
| | Mannitol | 5 g |
| | Polysorbate 80 | 0.1 g |
| | Sodium carboxymethyl cellulose | 0.5 g |
| | Sterile purified water | q.s. |

### Brief Description of the Drawing

[Fig. 1] Fig. 1 is a graph showing changes in intraocular pressure when betamethasone was incorporated in microspheres and administered to the sub-Tenon of cats.

## Claims

1. A method of relieving or avoiding a steroid-induced increase in intraocular pressure by incorporating a steroid in an ophthalmic composition in fine particles.

2. The method according to claim 1, wherein the site of administration is the sub-Tenon.

3. The method according to claim 2, wherein the dosage form of the ophthalmic composition is an injection.

4. The method according to claim 1, wherein an average particle diameter of the fine particles is 50 nm to 150 µm.

5. The method according to claim 4, wherein the fine particles are made of a biodegradable or biosoluble polymer.

6. The method according to claim 5, wherein the biodegradable or biosoluble polymer is polylactic acid or poly(lactic acid-glycolic acid).

7. The method according to claim 1, wherein the steroid is betamethasone, dexamethasone, triamcinolone, prednisolone, fluorometholone, hydrocortisone or fluocinolone acetonide.

8. An ophthalmic composition comprising a steroid, wherein a steroid-induced increase in intraocular pressure is relieved or avoided by incorporating the steroid in fine particles.

9. A method of relieving or avoiding a steroid-induced increase in intraocular pressure comprising administering an effective amount of an ophthalmic composition containing fine particles incorporating a steroid to a patient.

10. The method according to claim 9, wherein the site of administration is the sub-Tenon.

11. The method according to claim 10, wherein the dosage form of the ophthalmic composition is an injection.

12. The method according to claim 9, wherein an average particle diameter of the fine particles is 50 nm to 150 µm.

13. The method according to claim 12, wherein the fine particles are made of a biodegradable or biosoluble polymer.

14. The method according to claim 13, wherein the biodegradable or biosoluble polymer is polylactic acid or poly(lactic acid-glycolic acid).

15. The method according to claim 9, wherein the steroid is betamethasone, dexamethasone, triamcinolone, prednisolone, fluorometholone, hydrocortisone or fluocinolone acetonide.

16. Use of fine particles incorporating a steroid for production of an ophthalmic composition that relieves or avoids a steroid-induced increase in intraocular pressure.

17. The Use according to claim 16, wherein the site of administration is the sub-Tenon.

18. The Use according to claim 17, wherein the dosage form of the ophthalmic composition is an injection.

19. The Use according to claim 16, wherein an average particle diameter of the fine particles is 50 nm to 150 µm.

20. The Use according to claim 19, wherein the fine particles are made of a biodegradable or biosoluble polymer.

21. The Use according to claim 20, wherein the biodegradable or biosoluble polymer is polylactic acid or poly(lactic acid-glycolic acid).

22. The Use according to claim 16, wherein the steroid is betamethasone, dexamethasone, triamcinolone, prednisolone, fluorometholone, hydrocortisone or fluocinolone acetonide.
